# EUROPEAN PATENT APPLICATION

(11) **EP 3 586 930 A1**
(43) Date of publication of application: **01.01.2020**
(21) Application number: 17898241.9
(22) Date of filing: 21.02.2017
(51) Int. Cl.: A63B 71/12, A41B 11/00, A61F 13/08

(54) **SHIN GUARD SOCK FOR PRACTISING SPORT**

(71) Applicant: Rhinob Muscle Brand, S.L.U., 28600 Navalcarnero-Madrid (ES)
(72) Inventor: BARRIGÜETE DE GABRIEL, Carlos, 28600 Navalcarnero-Madrid (ES)
(74) Representative: Urizar Anasagasti, Jesus Maria
(86) International application number: PCT/ES2017/070092
(87) International publication number: WO 2018/154150

(57) **Abstract**

The invention relates to a shin guard sock for practicing sport, comprising, firstly, a sock (1) provided with a pocket-type compartment (2) on the inside, which is open at the top, in correspondence with the tibia, defining a space suitable for inserting a shin guard (3) therein, the sock being produced from a material comprising three protective layers: an internal layer (32) made of a thermoplastic polymer, such as EVA; an external layer (33) made of a polycarbonate; and an external coating (31) made of polyurethane elastomer sprayed onto the polycarbonate (33), forming an extremely rigid impact surface.

## Description

### Object of the invention

The present invention, as indicated by the title thereof, relates to a sock that has its own fastening and immobilising means for a shin guard, with the particular feature in that it also allows the material that makes up said shin guard to be a thermoplastic polymer, a material which has a greater ability to absorb blows, in addition to other advantages that will be explained in this description.

### Background of the invention

Currently, in the practice of sport such as football, weightlifting and other sports, it is necessary to use shin guards to protect athletes' tibias from blows by the weightlifting bars or by other football players. The majority of shin guards are rigid bodies, which are fastened to the leg by means of the sock itself, and thus they do not provide comfort or complete fastening, causing constant problems when the athlete is jumping, kicking, hitting against another object, running, etc., given that the protection moves from one side to another, up and down, or the shin guard comes out of the sock, and therefore, the majority of athletes use a Velcro strap or adhesive tape to fasten the shin guard to the leg, with the drawback in that it makes it even more uncomfortable for the athlete to move their leg while practicing the sport.

To prevent the mobility of the shin guard and to facilitate the placement thereof in a specific area of the leg, a sock has been designed which includes a front pocket into which the shin guard is inserted, this invention being described in document ES1081930, which relates to shin guard socks for practicing football, which incorporate an adjustable compartment into which the shin guard is inserted, situated in the area of the tibia with an opening at the top part of the compartment which can remain open, or if it is considered convenient, closing materials such as Velcro or other similar materials can be used to complement the immobilisation of the shin guard if necessary. Subsequently, this idea was also detailed in document ES1082305, which relates to a shin guard stabiliser that can be used for contact sports, which comprises an elastic tubular sock that has a front inner pocket intended to hold the shin guard, which is encapsulated in the pocket and adjusted by the elastic sock, provided with a Velcro closure on the front inner face of the pocket and another Velcro situated on the upper inner face of the sock which closes the compartment with the two closures, the first on the lower part of the sock made up of two rubber pieces, the lower rubber piece being adjustable with Velcro at the end thereof and the upper rubber piece adjustable with Velcro at the end thereof. On an international level there are other references to similar products, which also describe similar pockets, arranged on the front face of the sock, intended to hold the shin guard, such as documents WO201100662, GB2442190, GB2437242 and FR7730795. In principle, these fastening means should work correctly, but none of them has been put into practice, due to the simple fact that it is very difficult to make an inner pocket, joined to the sock.

With regard to the technology used in shin guards, specifically for use in practicing football and other similar sports in which impacts to the area of the tibia are common, it is important to highlight that, on the one hand, shin guards specifically used by professionals are known, which are even custom-made and made of carbon fibre or other very light yet resistant materials, while, on the other hand, more common shin guards are known, made of a polycarbonate-type plastic, which coats a padded inner area, and which can be made of ethylene-vinyl acetate (EVA) or a similar spongy and flexible material.

Another added problem is the fatigue accumulated in the legs of athletes, due to the amount of time they are on their feet, exercising intensely using that part of their body, leading on occasions to swelling of their legs and different types of muscular injuries. This can largely be avoided by using compression socks, but when shin guards are used below the same it is impossible to, first, place the socks over the shin guards, and second, keep the shin guards in position without them becoming bothersome to the athlete, since the pressure exerted by the additional fasteners of the shin guards usually causes discomfort.

### Description of the invention

Thus, the object of this model is a shin guard sock that combines the advantages of a sock with a pocket integrated on the front face of the same, which serves as a support and means for immobilising a light shin guard, but with a resistance to impact that is similar to other metal or carbon fibre shin guards, which once included in this pocket is kept in position, without the need to use complementary fastening means and which, therefore, provides a solution for many athletes who require an absolute fastening of their shin guards to their legs, also providing freedom of movement, comfort and a quick placement, while, in turn, eliminating the need to use adhesive tape.

The shin guard of the invention, which has been designed to be able to be included inside the pocket defined on the inside of the sock has three protective layers: an internal layer made of a thermoplastic polymer, such as ethylene-vinyl acetate, which is facing the leg, separated from it only by the fabric of the pocket that supports the shin guard; a second external layer made of a polycarbonate to which an external coating of polyurethane elastomer is applied by spraying, which is extremely hard and makes up the impact surface of the shin guard, and is, therefore, situated in contact with the inner face of the sock, in correspondence with the tibia of the leg on which it is placed. This coating forms a rough surface and therefore does not allow the shin guard to move once it is situated in position inside the pocket of the sock.

Furthermore, these types of shin guards and placement means made up of an inner pocket in the sock not only allow shin guards to be used in an effective and comfortable way, but the ideal use thereof is achieved with compression socks, which further allow for better circulation in athletes' legs, since they exert light pressure, allowing the blood to easily flow upwards, which helps prevent swelling of the legs, soreness and muscular injuries. The use of external compression socks causes different complex physiological and biochemical effects that affect the venous, arterial and lymphatic systems, while at the same time, regardless of the type of compression used, and if the right materials and techniques are applied, the effects of the compression can be highly beneficial, reducing swelling and pain as well as reducing problems caused by venous insufficiency.

The same inventor recently developed complementary fastening means for footwear made up of, among other elements, a series of prints on the support areas for the foot on the upper face of the insole of the shoe, which form a series of grooves and holes, with which the surface of the sock of the user makes contact, increasing the grip of the same and, therefore, of the foot, on the insole of the shoe. In a preferred embodiment, the sock of the invention has silicone prints on the lower part and external face thereof in support areas of the foot in the shoe, which, being equivalent to the ones on the insole of the shoe, also allow the foot to be perfectly secured inside the athlete's boot or shoe, preventing the movement thereof when there is a gap or when the corresponding fastening means are loosened.

### Description of the figures

As a complement to the present description, and for the purpose of helping to make the features of the invention more readily understandable, said description is accompanied by a set of drawings which, by way of illustration and not limitation, represent the following:

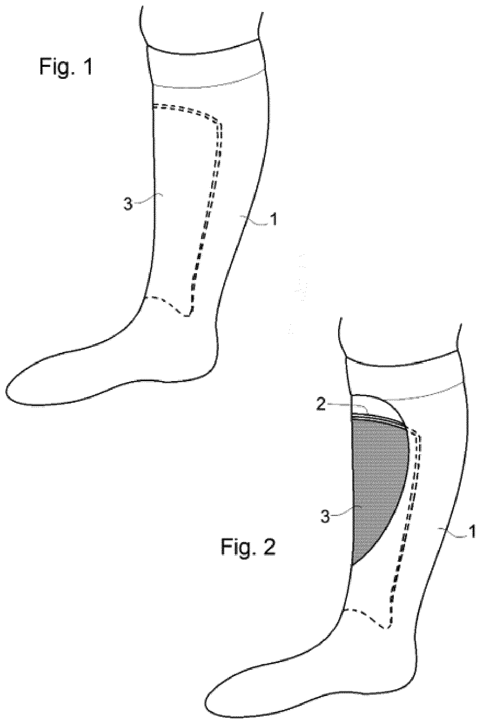

Figure 1 shows a general view of a sock (1) provided with a shin guard (3), which is fastened to the leg of an athlete by means of an inner pocket (2).
Figure 2 is a view that is equivalent to the previous figure, with a partial cross section in the front area so as to show the shin guard (3) and the pocket (2).
Figure 3 shows a view of the shin guard (3) outside of the sock (1).
Figure 4 shows a general view of a sock (1) provided with prints (12) on the sole of the foot.
Figure 5 represents a transverse cross-sectional view of a sock (1) at the central part of the shin guard (3) and the inner pocket (2).

### Preferred embodiment of the invention

As can be seen in the referenced figures, the shin guard sock for practicing sport object of this invention is of the type that incorporates a compartment (2) in the form of a pocket situated in the area of the tibia. This pocket (2) is open at the top and optionally provided with a type of closing means. According to an important feature of the invention, this pocket (2) is fastened by heat sealing to the front area of the sock (1), in correspondence with the athlete's tibia, and defines a space that is appropriate for inserting therein a shin guard (3) which remains immobilised in an operating position, protecting this area of the leg while the athlete is practicing sport.

In turn, the shin guard (3), which is inserted into the pocket (2) defined in the sock (1), has a constitution formed by three protective layers made of different materials; namely:
- an internal layer (32) made of a thermoplastic polymer, such as ethylene-vinyl acetate EVA, which is in contact with the compartment (2) on the inside of the sock (1);
- an external layer (33) made of a polycarbonate-type plastic; and
- an external coating (31) made of polyurethane elastomer sprayed onto the polycarbonate (33), which is in contact with the inner face of the sock (1) in the front area thereof, in correspondence with the tibia of the leg on which it is placed.

The external coating (31) is achieved by applying by means of spraying a layer of high-performance polyurethane elastomers, which do not contain volatile organic compounds (VOC) and which once applied make up a 100% solid product. In the tests carried out, LINE-X® was used, which is a product of this nature that offers an exceptional performance based on protective layers of elastomers for surfaces; this product is easy to use and fast drying (3 seconds), and furthermore, it offers exceptional adherence features and forms an excellent protective film for chemical resistance and as a protective barrier for moisture, among other features.

The sock (1) object of the invention is preferably a compressive elastic clothing item; with a composition of 85-95 % polyamide and 15-5% elastane, which is a synthetic fibre known for its exceptional elasticity, made up of a urethane-urea copolymer, which is extremely strong but not as long-lasting as natural latex.

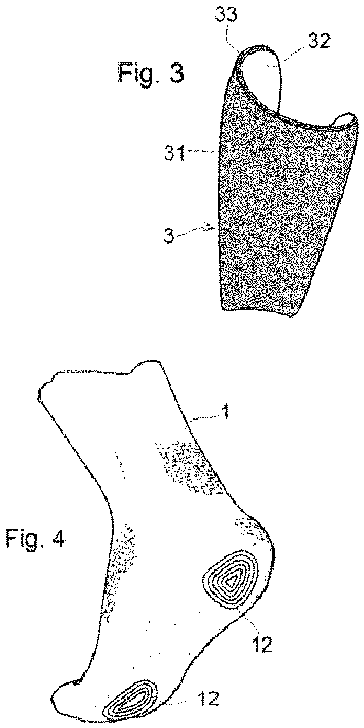

This sock (1), as can be seen in Figure 4, has silicone prints (12) on the lower part and outer face thereof in support areas of the foot in the shoe, which have the aim of preventing the mobility of the foot inside the shoe, when there is space between the two.

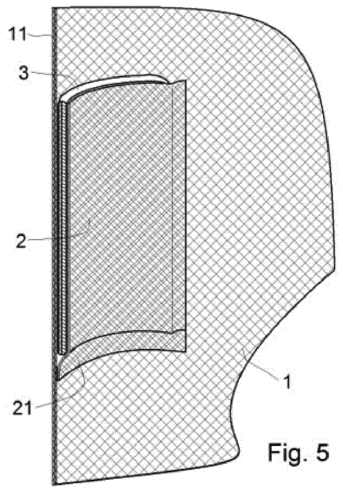

Figure 5 shows a cross section of a sock (1) with a compressive tissue (11) in which, on the front face thereof a pocket (29) has been heat sealed along perimeter strips (21), leaving the upper mouth open in order to allow a shin guard (3) of the mentioned type to be inserted in said pocket (2). The way of placing the same is extremely simple and without any added difficulty with respect to a conventional sock: once the sock (1) is put on and into position, below the knee, the shin guard (3) is inserted in the pocket defined in the front wall of the sock (1) and the heat sealed fabric on the inside, and once it slides to the bottom of said pocket (2) the sock is raised to its normal position, either below or above the knee. From that moment on, the sock is maintained in the final position thereof, and more easily when it is a compression sock; and for the same reason, the shin guard (3) is also maintained in the proper position, in front of the tibia and without moving to either side or downwards, given that the sock (1) along with the shin guard (3) prevents the same from happening.

Having sufficiently described the nature of the invention, in addition to an example of preferred embodiment, it is hereby stated for the relevant purposes that the materials, shape, size and layout of the described elements may be modified, provided that it does not imply altering the essential features of the invention claimed below:

## Claims

1. A shin guard sock for practicing sport, **comprising:**
- a sock (1) provided with a pocket-type compartment (2) on the inside, which is open at the top and which is made up of a fabric fixed by heat sealing to the front area of the sock (1), in correspondence with the athlete's tibia, and defines a space that is appropriate for inserting therein a shin guard (3) which remains immobilised in an operating position, protecting this area of the leg while the athlete is practicing sport; and
- a shin guard (3), which is situated inside the pocket (2) defined in the inside of the sock (1), which has three protective layers:
∘ an internal layer (32) made of a thermoplastic polymer, such as ethylene-vinyl acetate, which is in contact with the compartment (2) on the inside of the sock (1);
∘ an external layer (33) made of a polycarbonate; and
∘ an external coating (31) made of polyurethane elastomer sprayed onto the polycarbonate (33), which is in contact with the inner face of the sock (1) in the front area thereof, in correspondence with the tibia of the leg on which it is placed.

2. The shin guard sock for practicing sport, according to claim 1, **characterised in that** the sock (1) is a compressive elastic clothing item.

3. The shin guard sock for practicing sport, according to claim 2, **characterised in that** the compressive sock has a composition of 85--95 % polyamide and 15-5 % elastane.

4. The shin guard sock for practicing sport, according to any one of the preceding claims, **characterised in that** the sock (1) has silicone prints (12) on the lower part and outer face thereof in support areas of the foot in the shoe.
